(19) Europäisches Patentamt
European Patent Office
Office européen des brevets

(11) Publication number: **0 516 082 A2**

# (12) EUROPEAN PATENT APPLICATION

(21) Application number: **92108947.0**

(22) Date of filing: **27.05.92**

(51) Int. Cl.[5]: **C07C 49/245**, C07C 49/255, C07C 43/23, C07C 39/11, A61K 31/085, A61K 31/12

(30) Priority: **31.05.91 US 709012**

(43) Date of publication of application: **02.12.92 Bulletin 92/49**

(84) Designated Contracting States: **AT BE CH DE DK ES FR GB GR IT LI LU MC NL PT SE**

(71) Applicant: **MERRELL DOW PHARMACEUTICALS INC.**
**2110 East Galbraith Road**
**Cincinnati Ohio 45215(US)**

(72) Inventor: **Mao, Simon J.T.**
**9373 Kentonsrun Court**
**Loveland, Ohio 45140(US)**
Inventor: **King, Chi-Hsin R.**
**7381 Woodcroft Drive**
**Cincinnati, Ohio 45241(US)**

(74) Representative: **Vossius & Partner**
**Siebertstrasse 4 P.O. Box 86 07 67**
**W-8000 München 86(DE)**

(54) **Gingerol and gingerdiol derivatives as hypocholesterolemic and antiatherosclerotic agents.**

(57) The present invention relates to the use of a compound of the formula

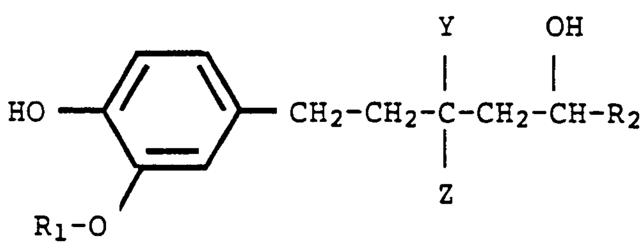

wherein $R_1$ is hydrogen or $C_1$-$C_4$ alkyl,
$R_2$ is a $C_1$-$C_{12}$ alkyl,
Y is O or OH, and
Z is a bond when Y is O, or is hydrogen when Y is OH
for the preparation of a pharmaceutical composition useful in lowering plasma cholesterol, inhibiting the progression of atherosclerosis and inhibiting the peroxidation of LDL lipid.

EP 0 516 082 A2

Atherosclerosis as manifested in its major clinical complication, ischaemic heart disease, continues to be a major cause of death in industrialized countries. It is now well accepted that atherosclerosis can begin with local injury to the arterial endothelium followed by proliferation of arterial smooth muscle cells from the medial to intimal layer along with deposition of lipid and accumulation of foam cells in the lesion. As the atherosclerotic plaque develops, it progressively occludes more and more of the affected blood vessel and can eventually lead to ischaemia or infarction. Therefore, it is desirable to provide pharmaceutical compositions useful in inhibiting the the progression of atherosclerosis in patients in need thereof.

There is now a large body of evidence demonstrating that hypercholesterolemia is an important risk factor associated with heart disease. For example, in December 1984, a National Institute of Health Consensus Development Conference Panel concluded that lowering definitely elevated blood cholesterol levels (specifically blood levels of low-density lipoprotein cholesterol) will reduce the risk of heart attacks due to coronary heart disease. Accordingly, it is desirable to provide a method for reducing plasma cholesterol in patients with hypercholesterolemia.

Typically, cholesterol is carried in the blood of warm-blooded animals in certain lipid-protein complexes such as chylomicrons, very low density lipoprotein (VLDL), low density lipoprotein (LDL), and high density lipoprotein (HDL). It is widely accepted that LDL functions in a way that directly results in deposition of the LDL cholesterol in the blood-vessel wall and that HDL functions in a way that results in the HDL reversely transporting cholesterol from the vessel wall to the liver where it is metabolized [Brown and Goldstein, *Ann.Rev.Biochem.* 52, 223 (1983); Miller, *Ann.Rev.Med.* 31, 97 (1980)]. For example, in various epidemiologic studies the LDL cholesterol levels correlate well with the risk of coronary heart disease whereas the HDL cholesterol levels are inversely associated with coronary heart disease [Mao et al., *Clin.Chem.* 29, 1890 (1983)]. It is generally accepted by those skilled in the art that reduction of abnormally high LDL cholesterol levels is effective therapy not only in the treatment of hypercholesterolemia but also in the treatment of atherosclerosis. Accordingly, it is desirable to provide a method for reducing LDL cholesterol in patients with hypercholesterolemia.

Furthermore, there is evidence based on animal and laboratory findings that peroxidation of LDL facilitates the accumulation of cholesterol in monocytes/macrophages which eventually transform into foam cells and deposit in the sub-endothelial space of the vessel wall. The accumulation of foam cells in the vessel wall is recognized as an early event in the formation of an atherosclerotic plaque. Thus it is believed that peroxidation of LDL is an important prerequisite to the facilitated accumulation of cholesterol in the vessel wall and the subsequent formation of an atherosclerotic plaque. For example, it has been shown that monocytes/macrophages take up and degrade native LDL at relatively low rates without marked accumulation of cholesterol. In contrast, oxidized LDL is taken up by these monocytes/macrophages at much higher rates with marked accumulation of cholesterol [See Parthasarathy et al., *J. Clin.Invest.* 77, 641 (1986)].

It has been shown, for example, that 2,2'-bis(3,5-ditertiary-butyl-4-hydroxyphenylthio)propane (also known as probucol), which is a known antioxidant, may prevent the progression of atherosclerosis in a manner which is independent of its effect on lowering plasma cholesterol levels [See Kita et. al., *Proc.Natl.Acad.Sci. USA* 84, 5928 (1987); Carew et al., *Proc.Natl.Acad.Sci. USA* 84, 7725 (1987); Mao et al., *J.Med.Chem.* 34, 298 (1991)]. It is believed that the antioxidant activity of probucol prevents atherogenesis by inhibiting lipid peroxidation of LDL. Accordingly, it is desirable to provide a method of inhibiting the lipid peroxidation of LDL.

The term "Gingerol" represents a homologous series of naturally-occurring phenolic ketones with the following structure:

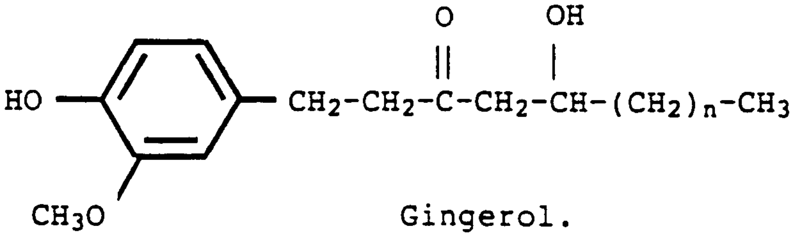

Gingerol.

The name [m]-gingerol is used for these compounds wherein m represents the chain length of the aldehyde formed in retroaldo cleavage. Thus [6]-gingerol, the major member of the series, has the above structure wherein n is 4. Denniff et al. [*J.Chem.Soc.Perkin Trans.* I, 2637 (1980); and *J.Chem.Soc. Perkin Trans.* I, 82 (1981)] have disclosed the existence of [3], [4], [5], [6], [8], [10], and [12]-gingerols. Denniff et al. (*Id.*)

have also disclosed that oleoresin contains [6], [8], and [10]-gingerdiols, however, the stereochemistry of the diols were not specified or identified. Gingerol is known to enhance gastrointestinal motility [Yamahara et al., *Chem.Pharm.Bull.* 38, 430 (1990)], to act as a cardiotonic [Kobayashi et al., *J.Pharm.Exp.Ther.* 246, 667 (1988)], to modulate eicosanoid-induced contraction of blood vessels [*Japan.J.Pharmacol.* 50, 253 (1989)], and to act as an analgesic and an antitussive.

[6]-Norgingerol, an analog of [6]-gingerol, is claimed in Japanese Patent J01066138, published March 13, 1989, as a new compound which inhibits 5-lipoxygenase. Norgingerol has the following structure:

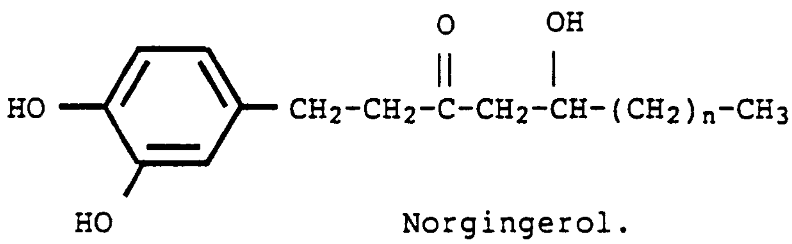

Norgingerol.

The present invention relates to the use of a compound of formula (I)

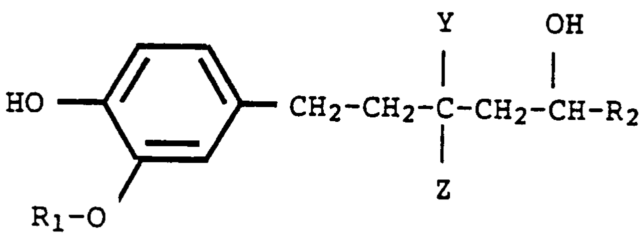

(I)

wherein $R_1$ is hydrogen or $C_1$-$C_4$ alkyl,

$R_2$ is a $C_1$-$C_{12}$ alkyl,

Y is O or OH, and

Z is a bond when Y is O, or is hydrogen when Y is OH for the preparation of a pharmaceutical composition useful in lowering plasma cholesterol.

The present invention also relates to the use of a compound of formula (I) for the preparation of a pharmaceutical composition useful in inhibiting the progression of atherosclerosis.

The present invention also relates to the use of a compound of formula (I) for the preparation of a pharmaceutical composition useful in inhibiting the peroxidation of LDL lipid.

The present invention also relates to novel compounds of the formula (II)

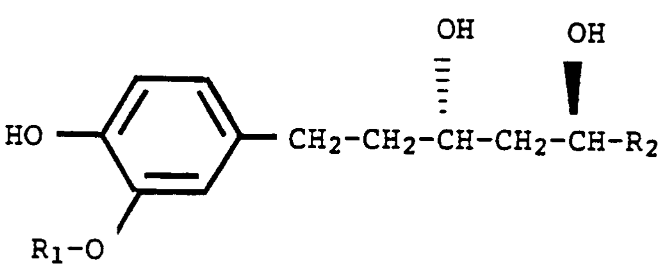

(II)

wherein $R_1$ is hydrogen or $C_1$-$C_4$ alkyl, and

$R_2$ is a $C_1$-$C_{12}$ alkyl.

As used herein, the term "$C_1$-$C_{12}$ alkyl" refers to a saturated hydrocarbyl radical of from 1 to 12 carbon atoms of straight, branched or cyclic configuration. Specifically included within the scope of the term are $-CH_3$, $-CH_2CH_3$, $-CH_2CH_2CH_3$, $-CH(CH_3)_2$, $-CH_2(CH_2)_2CH_3$, $-C(CH_3)_3$, $-CH_2CH(CH_3)_2$, $-CH_2(CH_2)_3CH_3$, $-CH_2(CH_2)_4CH_3$, $-CH_2(CH_2)_5CH_3$, $-CH_2(CH_2)_6CH_3$, $-CH_2(CH_2)_7CH_3$, $-CH_2(CH_2)_8CH_3$, $-CH_2(CH_2)_9CH_3$, $-CH_2$-

$(CH_2)_{10}CH_3$, cyclopropyl, cyclobutyl, cyclopentyl, cyclohexyl, cycloheptyl, cyclooctayl, cyclononyl, and the like.

As used herein, the term "$C_1$-$C_4$ alkyl" refers to a saturated hydrocarbyl radical of from 1 to 4 carbon atoms of straight, branched or cyclic configuration. Specifically included within the scope of the term are $-CH_3$, $-CH_2CH_3$, $-CH_2CH_2CH_3$, $-CH(CH_3)_2$, $-CH_2(CH_2)_2CH_3$, $-C(CH_3)_3$, $-CH_2CH(CH_3)_2$, cyclopropyl, cyclobutyl, and the like.

It is understood that compounds of formula (I) and (II) may exist in various stereoisomeric forms. All stereoisomeric forms which are consistent with the above structural formulas, as interpreted according to standard conventions for expressing stereoisomeric structure, are intended to be included within the scope of the present invention. For example, compounds of formula (I) wherein Y is hydroxy and Z is hydrogen, may exist with the aliphatic hydroxy groups being syn or anti to each other. Compounds of formula (II) however must bear aliphatic hydroxy groups which are anti to each other.

Gingerol derivatives of formula (I) may be prepared according to methods and procedures which are well known and appreciated in the art. For example, Denniff et al. [*J. Chem.Soc.,Chem.Comm.* 712 (1976); *J.Chem.Soc.,Perkin Trans.I,* 2637 (1980; and *J.Chem.Soc.,Perkin Trans.I,* 82 (1981)] disclose syntheses of various gingerols.

Alternatively, gingerol derivatives of formula (I) and anti-gingerdiol derivatives of formula (II) as well as syngingerdiol derivatives may be conveniently prepared as described in Scheme A. In Scheme A, all substituents, unless otherwise indicated are as previously defined.

## Scheme A

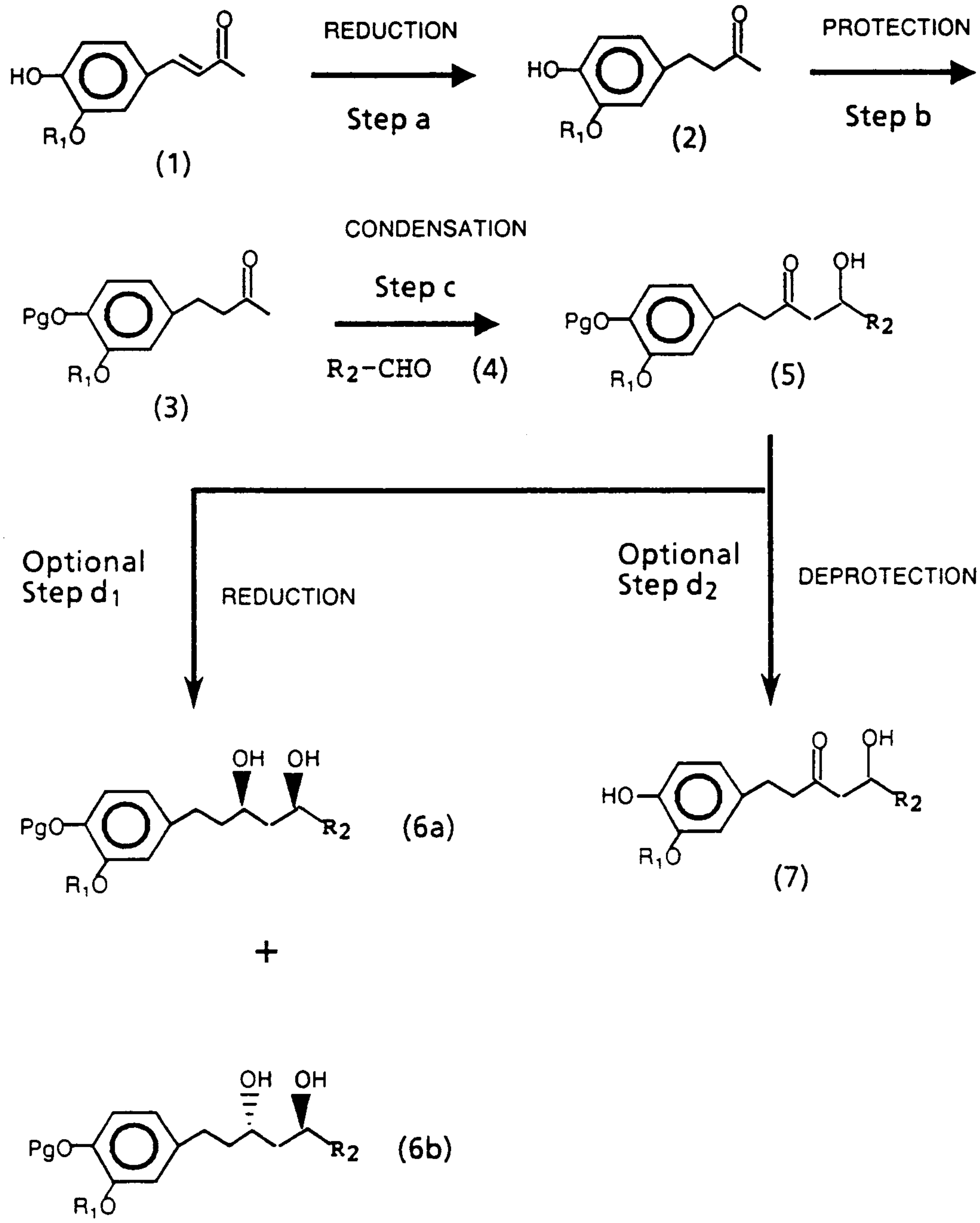


(1) (2) (3) (5) (6a) (6b) (7)

Scheme A Cont.

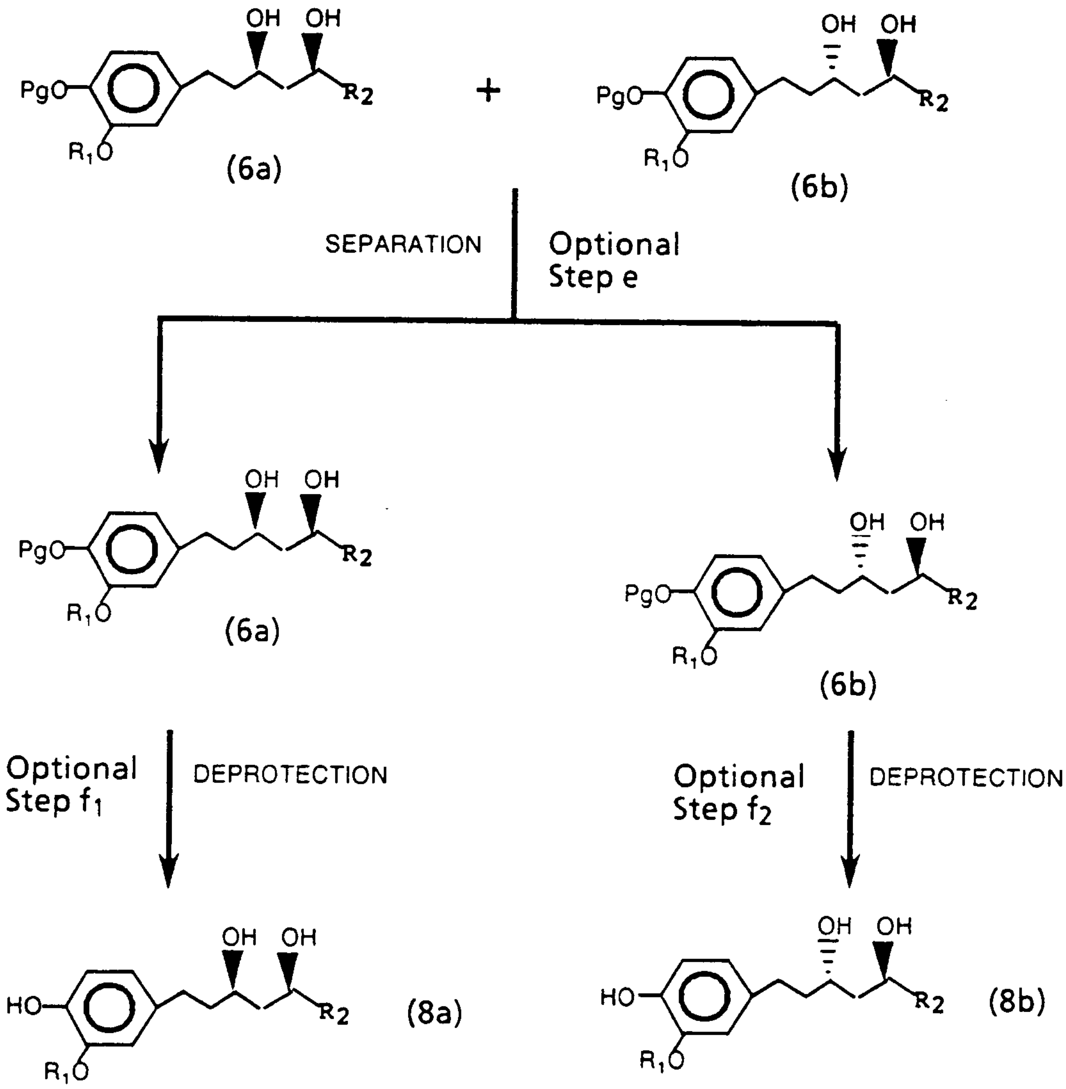


Scheme A provides a general synthetic procedure for preparing gingerol derivatives of formula (I) and formula (II).

In step a, the olefin functionality of the appropriate 4-(4'-hydroxyphenyl)-3-buten-2-one derivative of structure (1) is reduced to give the corresponding 4-(4'-hydroxyphenyl)-2-butanone derivative of structure (2).

For example, the appropriate 4-(4'-hydroxyphenyl)-3-buten-2-one derivative of structure (1) is contacted with a catalytic amount of a reducing agent such as Raney Nickel under a hydrogen atmosphere. The reactants are typically contacted in a suitable organic solvent such as acetone. The reactants are typically stirred together at room temperature for a period of time ranging from 2-24 hours. The 4-(4'-hydroxyphenyl)-2-butanone derivative of structure (2) is recovered from the reaction zone by filtration followed by evaporation of the solvent. It may be purified by silica gel chromatography.

In step b, the 4'-hydroxyphenyl functionality of the appropriate 4-(4'-hydroxyphenyl)-2-butanone derivative of structure (2) is protected to give the corresponding protected 4-(4'-hydroxyphenyl)-2-butanone derivative of structure (3). The selection and utilization of suitable protecting groups is well known by one of ordinary skill in the art and is described in "Protective Groups in Organic Syntheses", Theodora W. Greene, Wiley (1981). Examples of suitable protecting groups are benzyl ether, o-nitrobenzyl ether, 9-anthrylmethyl ether, 4-picolyl ether, and 4-methoxybenzyl ether wherein benzyl ether is preferred.

In step c, the appropriate protected 4-(4'-hydroxyphenyl)-2-butanone derivative of structure (3) is condensed with the appropriate aldehyde of structure (4) to give the corresponding protected gingerol of structure (5).

For example, the appropriate protected 4-(4'-hydroxyphenyl)-2-butanone derivative of structure (3) is first contacted with a slight molar excess of a suitable non-nucleophilic base such as lithium diisopropylamide. The reactants are typically contacted in a suitable anhydrous organic solvent such as tetrahydrofuran. The reactants are typically stirred together for a period of time ranging from 1-10 hours and at a temperature range of from -78°C to - 40°C. A slight molar excess of the appropriate aldehyde of structure (4) is then added. The reactants are typically stirred together for a period of time ranging from 5 minutes to 10 hours and at a temperature range of from -78°C to room temperature. The protected gingerol of structure (5) is recovered from the reaction zone by extractive methods as is known in the art. It may be purified by silica gel chromatography or recrystallization.

In optional step $d_1$, the ketone functionality of the appropriate protected gingerol derivative of structure (5) is reduced to give both the protected syn-gingerdiol of structure (6a) and the protected anti-gingerdiol of structure (6b).

For example, the appropriate protected gingerol derivative of structure (5) is contacted with a molar excess of a suitable reducing agent, such as sodium borohydride or tetramethylammonium triacetoxy borohydride. The reactants are typically contacted in a suitable organic solvent such as methanol or an acetic solvent mixture such as acetic acid/acetonitrile. The reactants are typically stirred together for a period of time ranging from 1-24 hours and at a temperature range of from -40°C to room temperature. The mixture of protected syn-gingerdiol of structure (6a) and protected anti-gingerdiol of structure (6b) are recovered from the reaction by extractive methods as is known in the art. The syn/anti composition is typically determined by high performance liquid chromatography (HPLC).

In optional step $d_2$, the appropriate protected gingerol derivative of structure (5) is deprotected to give the corresponding gingerol derivative of structure (7).

For example, the appropriate protected gingerol derivative of structure (5) is contacted with a catalytic amount of a suitable hydrogenation catalyst such as palladium/carbon under a hydrogen atmosphere. The reactants are typically contacted in a suitable organic solvent such as methanol. The reactants are typically shaken together at room temperature for a period of time ranging from 2-24 hours. The gingerol derivative of structure (7) is recovered from the reaction zone by filtration of the catalyst and evaporation of the solvent. It may be purified by silica gel chromatography.

In optional step e, the appropriate protected syngingerdiol of structure (6a) and the protected anti-gingerdiol of structure (6b) may be separated by techniques and procedures well known and appreciated by one of ordinary skill in the art such as recrystallization or chromatography.

In optional step $f_1$, the appropriate protected syn-gingerdiol of structure (6a) is deprotected to give the corresponding syn-gingerdiol derivative of structure (8a) as described previously in optional step $d_2$.

In optional step $f_2$, the appropriate protected anti-gingerdiol structure (6b) is deprotected to give the corresponding anti-gingerdiol derivative of structure (8b) as described previously in optional step $d_2$.

Alternatively, the mixture of the appropriate protected syn-gingerdiol of structure (6a) and the protected anti-gingerdiol of structure (6b) may be subjected to the deprotection conditions of optional steps $f_1$ and $f_2$, followed, if necessary, by the separation step of optional step e.

Starting materials for use in Scheme A are readily available to one of ordinary skill in the art.

The following examples represent typical syntheses as described in Scheme A. These examples are understood to be illustrative only and are not intended to limit the scope of the present invention in any way.

## Example 1

### 1-(4'-Hydroxy-3'-methoxyphenyl)-5-hydroxy-3-dodecanone ([8]-gingerol)

### Step a: 4-(4'-Hydroxy-3'-methoxyphenyl)-2-butanone

Dissolve vanillylacetone (400g, 2.08mol) in acetone (3L) and add Raney Ni (wet with water, about 40g) under a nitrogen atmosphere. Stir under a hydrogen atmosphere (20psi) at room temperature for 5 hours. Flush the reactor with nitrogen and filter to remove catalyst. Evaporate the solvent *in vacuo,* purify by silica gel chromatography (1:2ethyl acetate/hexane) and recrystallize (toluene) to give the title compound as a pale yellow solid (385g, 98%); mp 39-40°C.

IR (KBr) 3430 (br), 3002, 2940, 1712 (CO), 1640, 1516, 1466, 1452, 1432, 1366, 1272, 1236, 1216, 1154,

1124, 1034, 812, 794 $cm^{-1}$; $^1H$ NMR ($CDCl_3$) δ 6.9-6.6 (m, 3H), 5.57 (s, 1H), 3.87 (s, 3H), 2.8 (m, 2H), 2.7 (m, 2H), 2.12 (s, 3H); $^{13}C$ NMR ($CDCl_3$) δ 208.2, 146.4, 143.9, 132.9, 120.7, 114.3, 111.0, 55.8, 45.5, 30.1, 29.4; MS ($CI/CH_4$) m/z 195 ($MH^+$), 177, 165, 137.
Anal. Calcd for $C_{11}H_{13}O_3$: C, 68.02; H, 7.27;
Found: 67.95; H, 7.34.

Step b: 4-(4'-Benzyloxy-3'-methoxyphenyl)-2-butanone

Mix 4-(4'-hydroxy-3'-methoxyphenyl)-2-butanone (385g, 1.98mol), benzyl chloride (326g, 2.58mmol), potassium carbonate (274g, 1.98mol) and absolute ethanol (1.8L). Stir at reflux for 5 hours, filter the hot solution and evaporate the solvent *in vacuo.* Treat the residue with hexane (3L), stir at 4°C for 1 hour and filter to give the title compound as an off-white solid (465g, 82%); mp 60-62°C.
IR (KBr) 3440 (br), 3064, 3046, 3034, 2994, 2940, 1872, 1710, 1592, 1516, 1468, 1454, 1420, 1386, 1370, 1350, 1300, 1258, 1224, 1158, 1136, 1016, 926, 854, 804, 746, 700 $cm^{-1}$; $^1H$ NMR ($CDCl_3$) δ 7.5-7.2 (m, 5H), 6.8-6.6 (m, 3H), 5.12 (s, 2H), 3.86 (s, 3H), 2.8 (m, 2H), 2.7 (m, 2H), 2.13 (s, 3H); $^{13}C$ NMR ($CDCl_3$) δ 208.1, 149.5, 146.4, 137.3, 134.2, 128.5, 127.7, 127.2, 120.0, 114.2, 112.2, 71.1, 55.9, 45.3, 30.1, 29.3; MS ($CI/CH_4$) 285 (MH+), 267, 255, 227, 207, 91.
Anal. Calcd for $C_{18}H_{20}O_3$: C, 76.03; H, 7.09;
Found: C, 75.78; H, 7.10.

Step c: 1-(4'-Benzyloxy-3'-methoxyphenyl)-5-hydroxy-3-dodecanone

Dissolve diisopropylamine (156g, 1.54mol) in anhydrous tetrahydrofuran (1.4L). Cool to -70°C and place under a nitrogen atmosphere. Slowly add n-butyllithium (523mL of a 2.5M solution in hexane) over 30 minutes. Slowly add a solution of 4-(4'-benzyloxy-3'-methoxyphenyl)-2-butanone (365g, 1.28mol) in anhydrous tetrahydrofuran (1.4L) over 1 hour, keeping the temperature at -70°C. Stir the reaction mixture for 3 hours at -70°C and add n-octanal (202g, 1.58mol). Stir for 10 minutes and pour into a mixture of ethyl acetate (2L)/aqueous sodium hydrogen sulfate (1M, 3.8L). Stir for 20 minutes and separate the organic phase. Wash with saturated sodium hydrogen carbonate (1L) and dry ($MgSO_4$). Evaporate the solvent *in vacuo,* stir the residue with hexane (2L) for 18 hours and filter to give the title compound as a white solid (422g, 80%); mp 56-58°C.
IR (KBr) 3430 (br), 3050, 2956, 2924, 2854, 1706, 1606, 1590, 1516, 1468, 1454, 1416, 1380, 1258, 1228, 1156, 1142, 1094, 1030, 1010, 1000, 858, 808, 798, 746, 700 $cm^{-1}$; $^1H$ NMR ($CDCl_3$) δ 7.5-7.2 (m, 5H), 6.8-6.6 (m, 3H), 5.10 (s, 2H), 4.0 (m, 1H), 3.86 (s, 3H), 2.94 (s, 1H), 2.8 (m, 2H), 2.7 (m, 2H) , 2.5 (m, 2H), 1.5-1.2 (m 12H), 0.88 (t, 3H); $^{13}C$ NMR ($CDCl_3$) δ 211.3, 149.6, 146.6, 137.3, 134.0, 128.5, 127.7, 127.2, 120.0, 114,3, 112.2, 71.1, 67.6, 55.9, 49.3, 45.2, 36.5, 31.8, 29.5, 29.2, 25.4, 22.6, 14.0; MS ($CI/CH_4$) 413 (MH+), 412, 395, 269, 255, 227.
Anal. Calcd for $C_{26}H_{36}O_4$: C, 75.69; H, 8.80;
Found: C, 75.84; H, 8.96.

Optional Step $d_2$: 1-(4'-Hydroxy-3'-methoxyphenyl)-5-hydroxy-3-dodecanone ([8]-gingerol)

Place 10% palladium/carbon (0.5g, wet with 2mL of ethanol) in a hydrogenation flask under a nitrogen atmosphere. Add a solution of 1-(4'-benzyloxy-3'-methoxyphenyl)-5-hydroxy-3-dodecanone (4g, 0.01mol) in methanol (70mL). Hydrogenate at 55 psi at room temperature for 4 hours, filter and evaporate the solvent *in vacuo* to give the title compound as a colorless oil (3.1g, 99%).
IR (KBr) 3440 (br) , 2928, 2856, 1706, 1604, 1516, 1464, 1432, 1372, 1272, 1236, 1208, 1154, 1124, 1090, 1036, 852, 814, 798, 560 $cm^{-1}$; $^1H$ NMR ($CDCl_3$) δ 6.9-6.6 (m, 3H), 5.00 (s, 1H), 4.0 (m, 1H), 3.85 (s, 3H), 3.1 (m, 1H), 2.8 (m, 2H), 2.7 (m, 2H), 2.5 (m, 2H), 1.5-1.1 (m, 12H), 0.88 (t, 3H) ; $^{13}C$ NMR ($CDCl_3$) δ 49.3, 45.4, 36.4, 31.7, 29.6, 29.4, 29.2, 25.4, 22.6, 14.0; MS ($CI/CH_4$) 323 (MH+), 305, 179, 137.
HRMS: Found, 322.2141; Calcd for $C_{19}H_{30}O_4$, 322.2144.

Example 2

Anti-1-(4'-hydroxy-3'-methoxyphenyl)-3,5-dodecanediol (anti[8]-gingerdiol)

Optional Step $d_1$: Anti-1-(4'-benzyloxy-3'-methoxyphenyl)-3,5-dodecanediol and syn-1-(4'-benzyloxy-3'-methoxyphenyl)-3,5-dodecanediol

Add tetramethylammonium hydroxide (1.12L of a 25% solution in methanol, 3mol) and water (2L) to sodium borohydride (100g, 2.64mol). Stir for 15 minutes at room temperature and evaporate the solvent *in vacuo*. Suspend the solid white residue in 95% ethanol (1L), stir for 10 minutes and filter. Repeat this process five times and dry to give tetramethylammonium borohydride as a white solid (201g, 85%); mp 230-270 °C (dec. with gas evolution).

Slowly add tetramethylammonium borohydride (300g, 3.37mol) to cold stirring acetic acid (2.2L) over 2 hours under a nitrogen atmosphere. Stir at room temperature for 16 hours, add acetonitrile (2.2L) and filter. Add a solution of 1-(4'-benzyloxy-3'-methoxyphenyl)-5-hydroxy-3-dodecanone (230g, 0.56mol) in acetonitrile (0.8L) over 10 minutes at - 30 °C. Stir at 10 °C for 2 hours, quench with aqueous sodium potassium tartrate (1M, 2.5L) and stir at 0 °C for 20 minutes. Cool to -70 °C and treat with aqeuous sodium hydroxide (50%, 2.9kg) at a rate to maintain the internal temperature below 20 °C. Separate the organic phase from the solid by decantation, dissolve the solid in water (2L) and extract the resulting solution with ethyl acetate (2L). Combine the organic phases, wash with saturated sodium hydrogen carbonate (2X1L) and dry ($MgSO_4$). Evaporate the solvent *in vacuo* to give the title compounds as a semi solid (230g, 99%, anti/syn:3.5/1 by HPLC).

Recrystallize (ethyl ether) the mixture of anti-1-(4'-benzyloxy-3'-methoxyphenyl)-3,5-dodecanediol and syn-1-(4'-benzyloxy-3'-methoxyphenyl)-3,5-dodecanediol to give anti-1-(4'-benzyloxy-3'-methoxyphenyl)-3,5-dodecanediol; mp 83-85 °C.

IR (KBr) 3292 (br), 3062, 3034, 3004, 2932, 2914, 2852, 1602, 1586, 1516, 1466, 1452, 1416, 1378, 1320, 1266, 1246, 1224, 1186, 1158, 1136, 1106, 1076, 1056, 1034, 1002, 954, 944, 918, 908, 886, 854, 838, 808, 794, 748, 722 $cm^{-1}$; $^1H$ NMR ($CDCl_3$) $\delta$ 7.5-7.2 (m, 5H), 6.9-6.6 (m, 3H), 5.13 (s, 2H), 4.0 (m, 1H), 3.90 (s, 3H), 3.8 (m, 1H), 2.8-2.5 (m, 2H), 2.45 (s, 1H), 2.15 (s, 1H), 1.9-1.2 (m, 16H), 0.87 (t, 3H); $^{13}C$ NMR ($CDCl_3$) $\delta$ 149.6, 146.4, 137.4, 135.3, 128.5, 127.7, 127.2, 120.1, 114.3, 112.3, 71.2, 69.5, 68.9, 56.0, 42.4, 39.2, 37.5, 31.9, 31.8, 29.6, 29.2, 25.7, 22.6, 14.1; MS ($CI/CH_4$) 415 (MH+), 414, 398, 397, 379, 319, 289, 271, 255, 253, 228, 227, 163, 137, 91.

Anal. Calcd for $C_{26}H_{38}O_4$: C, 75.31; H, 9.24;

Found: C, 74.97; H, 9.06.

Optional step $f_2$: Anti-1-(4'-hydroxy-3'-methoxyphenyl)-3,5-dodecanediol (anti-[8]-gingerdiol)

Place 10% palladium/carbon (15g wet with 20 mL of ethanol) in a hydrogenation flask. Add a solution of anti-1-(4'-benzyloxy-3'-methoxyphenyl)-3,5-dodecanediol and syn-1-(4'-benzyloxy-3'-methoxyphenyl)-3,5-dodecanediol (220g, 0.53mol, anti/syn:3.5/1), in methanol (800mL) under a nitrogen atmosphere. Hydrogenate at 55 psi at room temperature for 5 hours. Filter the catalyst and evaporate the solvent in vacuo. Stir the oily residue with a mixture of ethyl ether (0.4L) and hexane (0.4L) for 18 hours. Filter to give the title compound as a white solid (116g, 67%); mp 94-96 °C

IR (KBr) 3298 (br) , 2956, 2922, 2850, 1606, 1518, 1468, 1456, 1446, 1408, 1368, 1274, 1230, 1150, 1120, 1100, 1070, 1030, 936, 820, 796, 722 620 $cm^{-1}$; $^1H$ NMR (DMSO-$d_6$) $\delta$ 8.60 (s, 1H), 6.8-6.5 (m, 3H), 4.32 (d, J=7.0 Hz, 1H), 4.20 (d, J-7.0 Hz, 1H), 3.74 (s, 3H), 3.6 (m, 2H), 2.7-2.5 (m, 2H), 1.6 (m, 2H), 1.4-1.1 (m, 14H), 0.86 (t, 3H); $^{13}C$ NMR (DMSO-$d_6$) $\delta$ 147.3, 144.3, 133.3, 120.2, 115.2, 112.5, 66.6, 66.2, 55.5, 44.9, 40.2, 38:0, 31.3, 31.1, 29.2, 28.8, 25.3, 22.1, 14.0; MS ($CJ/CH_4$) 325 (MH+), 324, 317, 307, 289, 207, 191, 177, 163, 151, 127, 123.

Anal. Calcd for $C_{19}H_{32}O_4$: C, 70.33; H, 9.94;

Found: C, 70.44; H, 9.89.

The following compounds can be prepared in a similar manner to that described in Examples 1 and 2:

1-(4'-Hydroxy-3'-ethoxyphenyl)-5-hydroxy-3-dodecanone;
1-(4'-Hydroxy-3'-ethoxyphenyl)-5-hydroxy-3-tetradecanone;
1-(4'-Hydroxy-3'-ethoxyphenyl)-5-hydroxy-3-hexadecanone;
1-(4'-Hydroxy-3'-butoxyphenyl)-5-hydroxy-3-dodecanone;
1-(4'-Hydroxy-3'-butoxyphenyl)-5-hydroxy-3-tetradecanone;
1-(4'-Hydroxy-3'-butoxyphenyl)-5-hydroxy-3-hexadecanone;
Anti-1-(4'-hydroxy-3'-ethoxyphenyl)-3,5-dodecanediol;
Anti-1-(4'-hydroxy-3'-ethoxyphenyl)-3,5-tetradecanediol;
Anti-1-(4'-hydroxy-3'-ethoxyphenyl)-3,5-hexadecanediol;
Anti-1-(4'-hydroxy-3'-butoxyphenyl)-3,5-dodecanediol;
Anti-1-(4'-hydroxy-3'-butoxyphenyl)-3,5-tetradecanediol;
Anti-1-(4'-hydroxy-3'-butoxyphenyl)-3,5-hexadecanediol;
Syn-1-(4'-hydroxy-3'-ethoxyphenyl)-3,5-dodecanediol;

Syn-1-(4'-hydroxy-3'-ethoxyphenyl)-3,5-tetradecanediol;
Syn-1-(4'-hydroxy-3'-ethoxyphenyl)-3,5-hexadecanediol;
Syn-1-(4'-hydroxy-3'-butoxyphenyl)-3,5-dodecanediol;
Syn-1-(4'-hydroxy-3'-butoxyphenyl)-3,5-tetradecanediol;
Syn-1-(4'-hydroxy-3'-butoxyphenyl)-3,5-hexadecanediol.

As used herein, the term "patient" refers to warm-blooded animals or mammals, including rabbits and humans, who are in need of treatment for atherosclerosis or hypercholesterolemia, such as, for example, in the case of a patient suffering from familial hyperlipidemia.

Hypercholesterolemia is a disease state characterized by levels of serum cholesterol or of LDL cholesterol which are elevated by a clinically significant amount over that considered normal by those of ordinary skill in the art. The identification of those patents who are in need of treatment for hypercholesterolemia is well within the ability and knowledge of one skilled in the art. For example, individuals who have serum cholesterol levels or LDL cholesterol levels, as determined by clinical laboratory tests, which are substantially and chronically elevated over that considered normal by those of ordinary skill in the art, are patients in need of treatment for hypercholesterolemia. By way of further example, individuals who are at risk of developing hypercholesterolemia can also be patients in need of treatment for hypercholesterolemia. A clinician skilled in the art can readily identify, by the use of clinical tests, physical examination and medical/family history, those patients who are suffering from hypercholesterolemia and those who are at risk of developing hypercholesterolemia and thus readily determine if an individual is a patient in need of treatment for hypercholesterolemia.

An effective hypocholesterolemic amount of a compound of formula (I) is an amount which is effective in reducing serum cholesterol levels or LDL cholesterol levels in a patient in need thereof. As such, successful treatment of a patient for hypercholesterolemia is understood to include reducing a patient's serum cholesterol or LDL cholesterol levels. Successful treatment for hypercholesterolemia is also understood to include prophylaxis in preventing clinically significant elevations in serum cholesterol or in LDL cholesterol levels in a patient who is at risk of the development of hypercholesterolemia.

Atherosclerosis is a disease state characterized by the development and growth of atherosclerotic lesions or plaque. The identification of those patients who are in need of treatment for atherosclerosis is well within the ability and knowledge of one skilled in the art. For example, individuals who are either suffering from clinically significant atherosclerosis or who are at risk of developing clinically significant atherosclerosis are patients in need of treatment for atherosclerosis. A clinician skilled in the art can readily determine, by the use of clinical tests, physical examination and medical/family history, if an individual is a patient in need of treatment for atherosclerosis.

An effective antiatherosclerotic amount of a compound of formula (I) is an amount which is effective in inhibiting development or growth of atherosclerosis in a patient in need thereof. As such, successful treatment of a patient for atherosclerosis is understood to include effectively slowing, interrupting, arresting, or stopping atherosclerotic lesion or plaque development or growth and does not necessarily indicate a total elimination of the atherosclerosis. It is further understood and appreciated by those skilled in the art that successful treatment for atherosclerosis can include prophylaxis in preventing atherosclerotic lesion or plaque formation.

It is believed that peroxidation of LDL facilitates the deposition of cholesterol in macrophages which subsequently are deposited in the vessel wall and are transformed into foam cells. The identification of those patients who are in need of inhibition of peroxidation of LDL is well within the ability and knowledge of one skilled in the art. For example, those individuals who are in need of treatment for atherosclerosis as defined hereinabove, are also patients who are in need of inhibition of peroxidation of LDL. Furthermore, an effective antioxidant amount of a compound of formula (I) is an amount which is effective in inhibiting the peroxidation of LDL in the patient's blood.

An effective antiatherosclerotic, antioxidant or hypocholesterolemic dose can be readily determined by the use of conventional techniques and by observing results obtained under analogous circumstances. In determining the effective dose, a number of factors are considered including, but not limited to: the species of patient; its size, age, and general health; the specific disease involved; the degree of or involvement or the severity of the disease; the response of the individual patient; the particular compound administered; the mode of administration; the bioavailability characteristics of the preparation administered; the dose regimen selected; and the use of concomitant medication.

An effective antiatherosclerotic, antioxidant or hypocholesterolemic amount of a compound of formula (I) will generally vary from about 0.1 milligram per kilogram of body weight per day (mg/kg/day) to about 5 grams per kilogram of body weight per day (gm/kg/day). A daily dose of from about 1 mg/kg to about 500 mg/kg is preferred.

The pharmaceutical composition containing a compound of formula (I) can be administered in any form or mode which makes the compound bioavailable in effective amounts, including oral and parenteral routes. For example, it can be administered orally, subcutaneously, intramuscularly, intravenously, transdermally, intranasally, rectally, and the like. Oral administration is generally preferred. One skilled in the art of preparing formulations can readily select the proper form and mode of administration depending upon the disease state to be treated, the stage of the disease, and other relevant circumstances.

A compound of formula (I) can be administered in the form of pharmaceutical compositions or medicaments which are made by combining the compound of formula (I) with pharmaceutically acceptable carriers or excipients, the proportion and nature of which are determined by the chosen route of administration, and standard pharmaceutical practice.

The pharmaceutical compositions or medicaments are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solution, suspensions, or the like.

The pharmaceutical compositions may be administered orally, for example, with an inert diluent or with an edible carrier. They may be enclosed in gelatin capsules or compressed into tablets. For the purpose of oral therapeutic administration, a compound of formula (I) may be incorporated with excipients and used in the form of tablets, troches, capsules, elixirs, suspensions, syrups, wafers, chewing gums and the like. These preparations should contain at least 4% of the compound of formula (I), the active ingredient, but may be varied depending upon the particular form and may conveniently be between 4% to about 70% of the weight of the unit. The amount of the active ingredient present in compositions is such that a unit dosage form suitable for administration will be obtained.

The tablets, pills, capsules, troches and the like may also contain one or more of the following adjuvants: binders, such as microcrystalline cellulose, gum tragacanth or gelatin; excipients, such as starch or lactose, disintegrating agents such as alginic acid, Primogel, corn starch and the like; lubricants, such as magnesium stearate or Sterotex; glidants, such as colloidal silicon dioxide; and sweetening agents, such as sucrose or saccharin may be added or flavoring agents, such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active ingredient, sucrose as a sweetening agent and certain preservatives, dyes and colorings and flavors. Materials used in preparing these various compositions should be pharmaceutically pure and non-toxic in the amounts used.

For the purpose of parenteral administration, a compound of formula (I) may be incorporated into a solution or suspension. These preparations should contain at least 0.1% of a compound of the invention, but may be varied to be between 0.1 and about 50% of the weight thereof. The amount of the active ingredient present in such compositions is such that a suitable dosage will be obtained.

The solutions or suspensions may also include one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of toxicity such as sodium chloride or dextrose. The parenteral preparation can be enclosed in ampules, disposable syringes or multiple dose vials made of glass or plastic.

The following examples illustrate the preparation and use of the compounds of formula (I) according to the present invention. These examples are illustrative only and are not intended to limit the scope of the invention in any way.

Example 3

Inhibition of LDL and Plasma Lipid Peroxidation

Determine the degree of inhibition of LDL and plasma lipid peroxidation by [8]-gingerol and anti-[8]-gingerdiol by the method of Yagi et al. [*Vitamins* 39, 105 (1968)].

Incubate a 0.5 mL solution containing 250 micrograms (µg) of LDL or 0.1 mL of plasma with the test compound, in amounts varying from 0 to 250 µg, for 30 minutes at 24° C. To this mixture add 10 µL of a

cupric sulfate solution (final concentration 18 µM in LDL assay, 2 mM in plasma assay) and incubate at 37°C for 2.5 hours or 18 hours. Determine the amount of peroxidation of LDL or plasma by the thiobarbituric acid assay. Calculate the dose of test compound required to inhibit 50% of LDL or plasma lipid peroxidation ($ID_{50}$) and the concentration of test compound required to inhibit 50% of LDL or plasma lipid peroxidation ($IC_{50}$). Results are presented in Table 1.

Table 1

| Effect of [8]-Gingerol and anti-[8]-Gingerdiol on LDL and Plasma Lipid Peroxidation | | | | |
|---|---|---|---|---|
| Treatment | LDL Perox. $ID_{50}$ | LDL Perox. $IC_{50}$ | Plas.Lipid Perox. $ID_{50}$ | Plas.Lipid Perox. $IC_{50}$ |
| [8]-Gingerol[a] | 60 nmole | 109 µM | 5 nmole | 8.3 µM |
| anti-[8]-Gingerdiol[b] | 40 nmole | 72 µM | 10 nmole | 16.6µM |

a[8]-Gingerol is 1-(4'-hydroxy-3'-methoxyphenyl)-5-hydroxy-3-dodecanone
[b]anti-[8]-Gingerdiol is anti-1-(4'-hydroxy-3'-methoxyphenyl)-3,5-dodecanediol

Example 4

Effect of anti-[8]-Gingerdiol on Serum Cholesterol in the Cholesterol-Fed Rabbit

Feed rabbits with 1% cholesterol (w/w) in standard rabbit chow supplemented with (n=5) or without (n=8) 0.5% anti-[8]-gingerdiol (w/w). Each rabbit consumes 120 grams of food per day. Collect blood samples and determine cholesterol in freshly prepared serum using an enzymatic method described by Mao et al., *Clin.Chem* 29, 1890 (1983). Results are presented in Table 2.

Example 5

Antiatherogenic Effect of anti-[8]-Gingerdiol in the Cholesterol-Fed Rabbit

Feed rabbits with 1% cholesterol (w/w) in standard rabbit chow supplemented with (n=5) or without (n=6) 0.5% anti-[8]-gingerdiol (w/w) for 56 days. Each rabbit consumes 120 grams of food per day. Immediately following sacrifice by intravenous injection of sodium pentobarbital, dissect the aortas from the ascending arch to the ileal bifurcation.

Table 2

| Effect of anti-[8]-Gingerdiol on Serum Cholesterol in the Cholesterol-Fed Rabbit | | | |
|---|---|---|---|
| Treatment | Cholesterol (mg/dL±SEM) Day 0 | Cholesterol (mg/dL±SEM) Day 10 | Cholesterol (mg/dL±SEM) Day 28 |
| Control (n=8) | 72 ± 13 | 790 ± 103 | 2177 ± 97 |
| 0.5% anti-[8]-Gingerdiol[a] (n=5) | 82 ± 12 | 515 ± 154 | 1615 ± 216 |

[a]anti-[8]-Gingerdiol is anti-1-(4'-hydroxy-3'-methoxyphenyl)-3,5-dodecanediol

Remove extraneous adipose tissue, open the aortas longitudinally and rinse several times with saline. Stain the aortas with Sudan IV and photograph using the method described by Mao et al. [*J.Med.Chem.* 34, 302 (1991)]. Digitize the areas of sudanophilic lesions in the thoracic region of the aorta with a Magiscan image analysis system. Distinguish stained versus unstained areas on the basis of Sudan Red staining. Quantitate the corresponding lesioned and unlesioned areas accordingly. Results are presented in Table 3.

Table 3

| Antiatherogenic Effect of anti-[8]-Gingerdiol in the Cholesterol-Fed Rabbit | | | |
|---|---|---|---|
| Treatment | % Lesions (±SEM) in Arch | % Lesions (±SEM) in Thoracic | % Lesions (±SEM) in Abdominal |
| Control (n=6) | 83 ± 7 | 62 ± 8 | 28 ± 7 |
| 0.5% anti-[8]-Gingerdiol[a] (n=5) | 73 ± 12 | 38 ± 16 | 24 ± 10 |

[a]anti-[8]-Gingerdiol is anti-1-(4'-hydroxy-3'-methoxyphenyl)-3,5-dodecanediol

As with any group of structurally related compounds having a common property, some substituents and configurations of the compounds are preferred. In the method of use of the present invention, the following substituents are preferred: compounds wherein $R_1$ is methyl; compounds wherein $R_2$ is heptyl; compounds wherein Y is hydroxy and Z is hydrogen; and compounds wherein Y is hydroxy and is in an *anti* position with respect to the other aliphatic hydroxy group.

## Claims

**1.** Use of a compound of the formula (I)

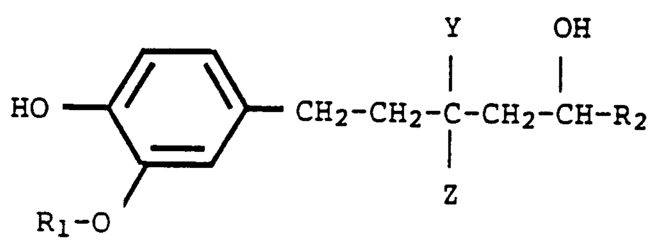

wherein $R_1$ is hydrogen or $C_1$-$C_4$ alkyl,
$R_2$ is a $C_1$-$C_{12}$ alkyl,
Y is O or OH, and
Z is a bond when Y is O, or is hydrogen when Y is OH for the preparation of a pharmaceutical composition useful in lowering plasma cholesterol.

**2.** Use of the compounds as defined in Claim 1 for the preparation of a pharmaceutical composition useful in inhibiting the progression of atherosclerosis.

**3.** Use of the compounds as defined in Claim 1 for the preparation of a pharmaceutical composition useful in inhibiting the peroxidation of LDL lipid.

**4.** Use according to Claim 1, 2 or 3 wherein in the compound $R_1$ is methyl.

**5.** Use according to Claim 1, 2 or 3 wherein in the compound $R_2$ is n-heptyl.

**6.** Use according to Claim 1, 2 or 3 wherein in the compound Y is OH and Z is hydrogen.

**7.** Use according to Claim 1, 2 or 3 wherein in the compound Y is O and Z is a bond.

**8.** Use according to Claim 1, 2 or 3 wherein the compound is 1-(4'-hydroxy-3'-methoxyphenyl)-5-hydroxy-3-dodecanone.

**9.** Use according to Claim 1, 2 or 3 wherein the compound is anti-1-(4'-hydroxy-3'-methoxyphenyl)-3,5-dodecanediol.

**10.** A compound of the formula

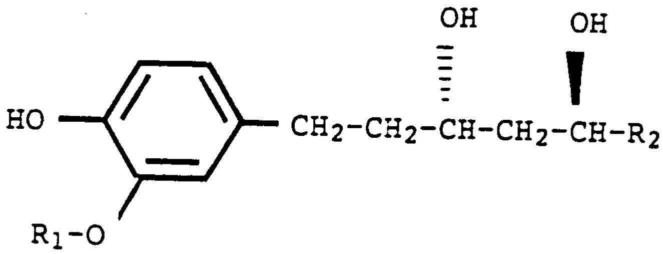

wherein $R_1$ is hydrogen or $C_1$-$C_4$ alkyl, and
$R_2$ is a $C_1$-$C_{12}$ alkyl.

**11.** A compound according to Claim 10 wherein the compound is anti-1-(4'-hydroxy-3'-methoxyphenyl)-3,5-dodecanediol.

**Claims for the following Contracting State : GR**

**1.** A method for the preparation of a pharmaceutical composition useful in lowering plasma cholesterol comprising combining a compound of the formula

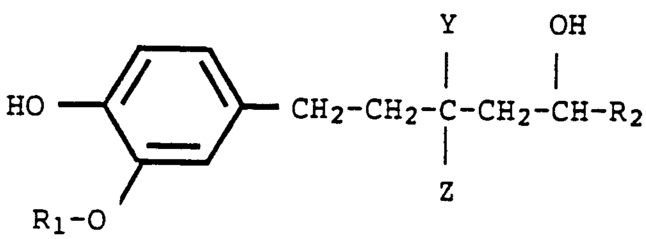

wherein $R_1$ is hydrogen or $C_1$-$C_4$ alkyl,
$R_2$ is a $C_1$-$C_{12}$ alkyl,
Y is O or OH, and
Z is a bond when Y is O, or is hydrogen when Y is OH

with a pharmaceutically acceptable carrier.

**2.** A method for the preparation of a pharmaceutical composition useful in inhibiting the progression of atherosclerosis comprising combining a compound as defined in Claim 1 with a pharmaceutically acceptable carrier.

**3.** A method for the preparation of a pharmaceutical composition useful in inhibiting the peroxidation of LDL lipid comprising combining a compound as defined in Claim 1 with a pharmaceutically acceptable carrier.

**4.** A method according to Claim 1, 2 or 3 wherein in the compound used $R_1$ is methyl.

**5.** A method according to Claim 1, 2 or 3 wherein in the compound used $R_2$ is n-heptyl.

**6.** A method according to Claim 1, 2 or 3 wherein in the compound used Y is OH and Z is hydrogen.

**7.** A method according to Claim 1, 2 or 3 wherein in the compound used Y is O and Z is a bond.

**8.** A method according to Claim 1, 2 or 3 wherein the compound used is 1-(4'-hydroxy-3'-methoxyphenyl)-5-hydroxy-3-dodecanone.

**9.** A method according to Claim 1, 2 or 3 wherein the compound used is anti-1-(4'-hydroxy-3'-methoxyphenyl)-3,5-dodecanediol.

**10.** A compound of the formula

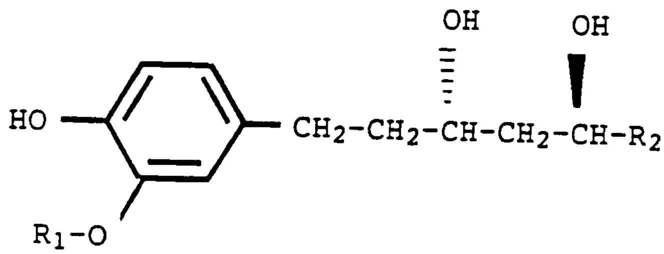

wherein $R_1$ is hydrogen or $C_1$-$C_4$ alkyl, and
$R_2$ is a $C_1$-$C_{12}$ alkyl.

**11.** A compound according to Claim 10 wherein the compound is anti-1-(4'-hydroxy-3'-methoxyphenyl)-3,5-dodecanediol.

**Claims for the following Contracting State : ES**

**1.** A method for the preparation of a pharmaceutical composition useful in lowering plasma cholesterol comprising combining a compound of the formula

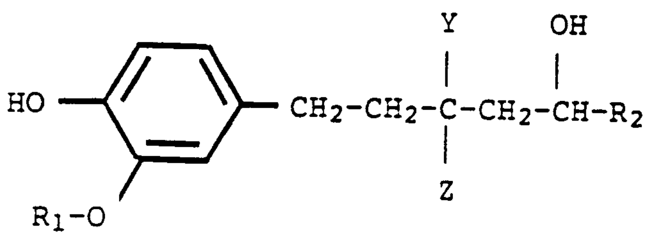

wherein $R_1$ is hydrogen or $C_1$-$C_4$ alkyl,
$R_2$ is a $C_1$-$C_{12}$ alkyl,
Y is O or OH, and
Z is a bond when Y is O, or is hydrogen when Y is OH

with a pharmaceutically acceptable carrier.

**2.** A method for the preparation of a pharmaceutical composition useful in inhibiting the progression of atherosclerosis comprising combining a compound as defined in Claim 1 with a pharmaceutically acceptable carrier.

**3.** A method for the preparation of a pharmaceutical composition useful in inhibiting the peroxidation of LDL lipid comprising combining a compound as defined in Claim 1 with a pharmaceutically acceptable carrier.

**4.** A method according to Claim 1, 2 or 3 wherein in the compound used $R_1$ is methyl.

**5.** A method according to Claim 1, 2 or 3 wherein in the compound used $R_2$ is n-heptyl.

**6.** A method according to Claim 1, 2 or 3 wherein in the compound used Y is OH and Z is hydrogen.

**7.** A method according to Claim 1, 2 or 3 wherein in the compound used Y is O and Z is a bond.

**8.** A method according to Claim 1, 2 or 3 wherein the compound used is 1-(4'-hydroxy-3'-methoxyphenyl)-5-hydroxy-3-dodecanone.

**9.** A method according to Claim 1, 2 or 3 wherein the compound used is anti-1-(4'-hydroxy-3'-methoxyphenyl)-3,5-dodecanediol.

**10.** A process for preparing compounds of the formula

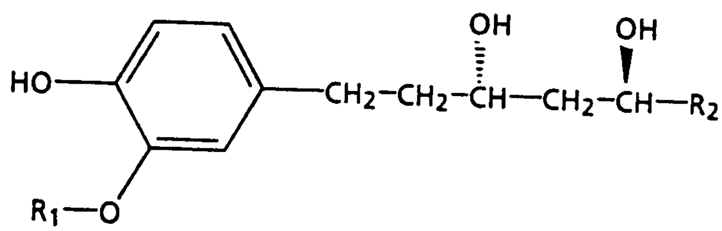

wherein

$R_1$ is hydrogen or $C_1$-$C_4$ alkyl, and

$R_2$ is a $C_1$-$C_{12}$ alkyl comprising separating the protected anti diol from a mixture of protected syn and anti diol, and subsequently hydrogenolyzing the anti diol of formula

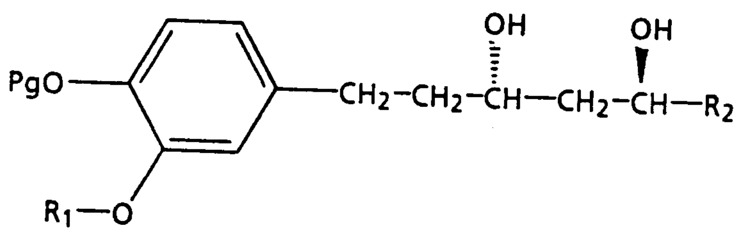

in which $R_1$ and $R_2$ are defined as above and Pg is a suitable protecting group.

**11.** A process according to Claim 10 wherein the compound prepared is anti-1-(4'-hydroxy-3'-methoxyphenyl)-3,5-dodecanediol.